(19) Europäisches Patentamt
European Patent Office
Office européen des brevets

(11) Veröffentlichungsnummer: **0 009 186**
**A1**

(12) # EUROPÄISCHE PATENTANMELDUNG

(21) Anmeldenummer: **79103332.7**

(22) Anmeldetag: **07.09.79**

(51) Int. Cl.³: **C 07 C 123/00,** C 08 F 4/04, C 08 J 9/10, C 08 K 5/31

(30) Priorität: **21.09.78 DE 2841045**
**21.09.78 DE 2841046**

(43) Veröffentlichungstag der Anmeldung: **02.04.80 Patentblatt 80/7**

(84) Benannte Vertragsstaaten: **BE DE FR GB IT NL**

(71) Anmelder: **BAYER Aktiengesellschaft, Zentralbereich Patente,Marken und Lizenzen Bayerwerk, D-5090 Leverkusen 1 (DE)**

(72) Erfinder: **Roos, Ernst, Dr., Am Gensgarten 6, D-5068 Odenthal (DE)**
Erfinder: **Bartl, Herbert, Dr., Eichendorffweg 10, D-5068 Odenthal (DE)**
Erfinder: **Schuster, Klaus, Gerhart-Hauptmann-Strasse 33, D-5090 Leverkusen 3 (DE)**
Erfinder: **Schmidt, Adolf, Dr., Roggendorfstrasse 67, D-5000 Köln 80 (DE)**

(54) **Azo-di-isobuttersäure-(N,N'-hydroxialkyl)-amidine, ihre Herstellung und Verwendung als Polymerisationsinitiatoren, als Vernetzungsmittel und als Treibmittel.**

(57) Die Erfindung betrifft Azo-di-isobuttersäure-(N,N'-hydroxialkyl)-amidine der Formel

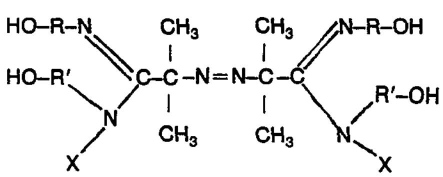

worin
R und R' lineare oder verzweigte Alkylenreste mit 2 bis 4 C-Atomen, und
X R'–OH oder H bedeuten;
ein Verfahren zu ihrer Herstellung, sowie ihre Verwendung als Polymerisationsinitiatoren, als Vernetzungsmittel und als Treibmittel bei der Herstellung von Schaumstoffen.

EP 0 009 186 A1

BAYER AKTIENGESELLSCHAFT
Zentralbereich
Patente, Marken und Lizenzen

5090 Leverkusen, Bayerwerk

Fr-by

Azo-di-isobuttersäure-(N,N'-hydroxialkyl)-amidine, ihre Herstellung und Verwendung als Polymerisationsinitiatoren, als Vernetzungsmittel und als Treibmittel

Die Erfindung betrifft neue Azo-di-isobuttersäure-(N,N'-hydroxialkyl)-amidine und deren Verwendung als Polymerisationsinitiatoren, als Vernetzungsmittel und Treibmittel bei der Herstellung von Schaumstoffen.

Aus der US-Patentschrift 2 599 299 ist es bekannt, das Dihydrochlorid des Azo-di-isobuttersäureamidins herzustellen.

Aus der DE-AS 1 693 164 ist ferner bekannt, auch das säurefreie Azo-di-isobuttersäureamidin herzustellen, wobei allerdings besondere Vorsichtsmaßnahmen getroffen werden müssen, damit sich das wasserfeuchte Produkt nicht zersetzt.

Auch die Verwendung dieser Verbindungen als Polymerisationsinitiatoren wurde bereits beschrieben (vgl. US-PS 2 599 300).

Sie konnte jedoch wegen der Instabilität der Initiatoren selbst sowie wegen der Korrosions- und Koagulationsprobleme, die bei deren Verwendung als Initiatoren auftreten, technisch bisher nicht genutzt werden. Hierfür ist insbesondere die Hydrolyse der freien Amidingruppe, die zu Ammoniak, Amidgruppen und Ammoniumsalzgruppen führt, verantwortlich:

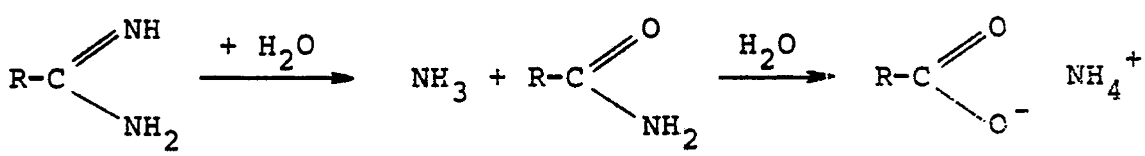


R = Rest des Initiatormoleküls

Während nämlich die Wasserlöslichkeit der genannten Radikalbildner für die Polymerisation in wäßrigen Suspensionen oder Emulsionen an sich eine sehr erwünschte Eigenschaft darstellt, stört in vielen Fällen das Auftreten von Salzen den Polymerisationsverlauf. Bei empfindlichen Emulsionen kann es dadurch zum vorzeitigen, unerwünschten Koagulieren der Emulsionen kommen. Andererseits stört in vielen Fällen auch der Einbau von salzartigen Gruppen in das Polymerisat und kann deren Eigenschaften sehr nachteilig beeinflussen.

Überraschenderweise wurde nun gefunden, daß man durch Verwendung der neuen Azo-di-isobuttersäure-(N,N'-hydroxialkyl)-amidine der Formel (I)

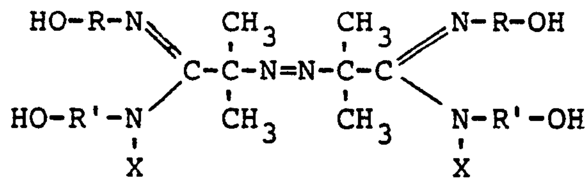

(I)

in welcher

R und R' lineare oder verzweigte Alkylenreste mit 2 bis 4 Kohlenstoffatomen bedeuten und

X für Wasserstoff oder -R'-OH steht,

als Polymerisationsinitiatoren die obengenannten Nachteile vermeidet. Darüber hinaus erhält man Polymerisate, die durch den Einbau der hydrophilen Hydroxialkylgruppen ganz spezielle und erwünschte Eigenschaften aufweisen.

Vorzugsweise bedeuten in Formel (I) R und R' lineare oder verzweigte Alkylenreste mit 2 bis 3 C-Atomen wie $-CH_2-CH_2-$, $-CH_2-CH_2-CH_2-$ oder

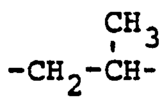

und X Wasserstoff oder R'-OH.

Insbesondere ist in Formel (I) R=R', bedeuten R und R' einen linearen Alkylenrest mit 2 C-Atomen (=Ethylenrest) und steht X für Wasserstoff oder ß-Hydroxiethylrest.

Die Azo-di-isobuttersäure-(N,N'-hydroxialkyl)-amidine der Formel (I) sind Gegenstand der Erfindung, ebenso ihre Verwendung als Radikalbildner für Polymerisationsprozesse ungesättigter Verbindungen und/oder Vernetzungsprozesse mehrfach ungesättigter polymerisierbarer Verbindungen.

Die Herstellung der erfindungsgemäßen Azo-di-isobuttersäure-(N,N'-bis-hydroxialkyl)-amidine und Azo-di-isobuttersäure-(N,N'-tris-hydroxialkyl)-amidine kann dadurch erfolgen, daß man

A) An den N-Atomen nicht-substituiertes Azo-di-isobuttersäureamidin oder an den N-Atomen durch einen bis fünf Hydroxialkylreste mit 2-4 C-Atomen substituiertes Amidin mit Alkylenoxiden ($C_2$ - $C_4$) umsetzt oder

B) Azo-di-isobuttersäure-iminoalkylether der allgemeinen Formel (II) mit Monoalkanolaminen oder Mischungen aus Monoalkanol- und Di-alkanolaminen umsetzt.

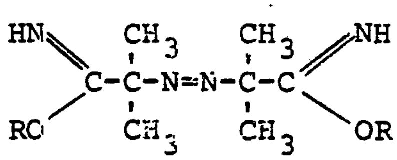
(II)

In der Formel (II) bedeutet R niedere Alkylreste mit 1-4 C-Atomen.

Beispielhaft sei die Umsetzung von Azo-di-isobuttersäure-iminomethylether mit Monoethanolamin [Formelschema (IIIa)] und die Umsetzung von Azo-di-isobuttersäure-iminoethylether mit einem Gemisch aus Mono- und Diethanolamin [Formelschema (IIIb)] beschrieben:

(IIIa)

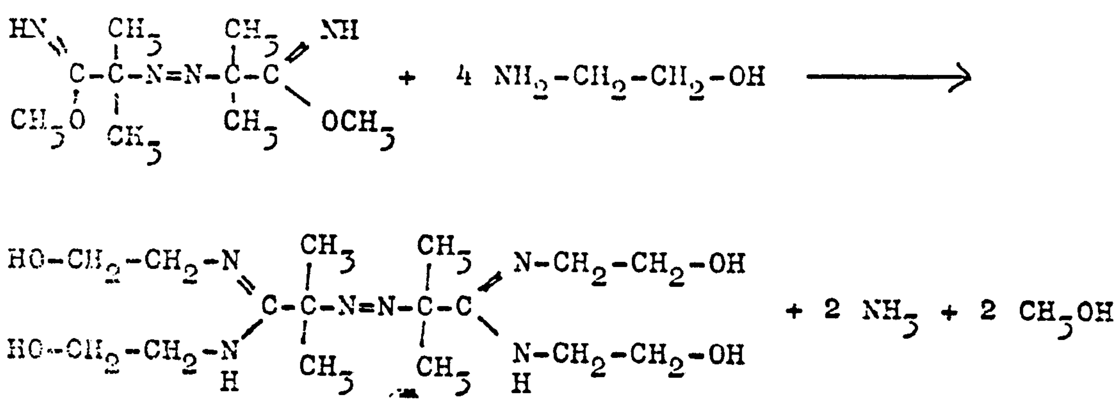

(IIIb)

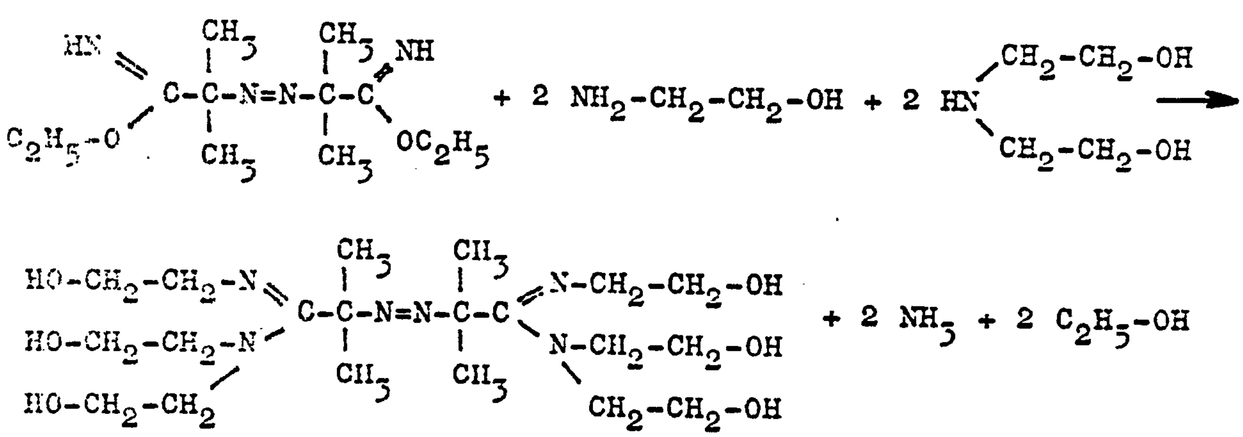

Es ist ohne weiteres möglich, Azo-di-isobuttersäure-(N,N'-bis- bzw. N,N'-tris-hydroxialkyl)amidine dadurch herzustellen, daß man Azo-di-isobuttersäureamidin zunächst teilweise mit einen Alkylenoxid umsetzt und anschließend bis zum gewünschten Substitutionsgrad mit Mono- und/oder Dialkanolamin kondensiert bzw. die umgekehrte Reihenfolge wählt. Andererseits können die Iminogruppen der Azo-di-isobuttersäure-iminoalkylether zunächst ganz oder teilweise mit einem Alkylenoxid umgesetzt werden und anschließend die Alkylethergruppen und gegebenenfalls restliche Iminogruppen mit Mono- und/oder Dialkanolamin bis zum N,N'-Bis-(hydroxialkyl)- bzw. N,N',N'-Tris-(hydroxialkyl)-amidin der Azo-di-isobuttersäure kondensiert werden.

Vorzugsweise werden die Azo-di-isobuttersäure-(N,N'-bis-bzw. N,N',N'-tris-hydroxialkyl)-amidine durch Umsetzung der Azo-di-isobuttersäure-iminoalkylether mit Monoalkanolaminen oder mit Mischungen aus Monoalkanol- und Dialkanolaminen (Molverhältnis 1:1) hergestellt.

Die Anlagerungsreaktion mit den Alkylenoxiden bzw. die Kondensation mit Mono- und/oder Dialkanolamin erfolgt bei 0-50°C, vorzugsweise 20-45°C. Die Reaktionen können lösungsmittelfrei oder in Gegenwart von organischen Lösungsmitteln erfolgen, die unter den Versuchsbedingungen indifferent gegenüber den Reaktionsteilnehmern sind, beispielsweise in Alkoholen wie Methanol, Ethanol, in Ethern wie Diethylether oder Dioxan; in Ketonen wie Aceton oder Ethylmethylketon, aber auch in aliphatischen

oder aromatischen Kohlenwasserstoffen. Die Reaktionen können drucklos oder unter Drücken bis 50 bar durchgeführt werden.

Als Alkylenoxide kommen in Frage: Ethylenoxid, Propylenoxid, 1,2-Epoxibutan, 2,3-Epoxibutan, 1,2-Epoxi-2-methylpropan, vorzugsweise Ethylenoxid und Propylenoxid, insbesondere Ethylenoxid.

Für die Umsetzung mit den Iminoalkylethern können eingesetzt werden: Ethanolamin, Diethanolamin, 1-Amino-propanol-(2), Bis-(2-hydroxi-propyl)-amin; 1-Amino-propanol-(3), Bis-(3-hydroxi-propyl)-amin; Isopropanolamin, Diisopropanolamin; 1-Amino-butanol(4), Bis-(4-hydroxi-butyl)-amin; 1-Amino-butanol-(3), Bis-(3-hydroxi-butyl)-amin; 1-Amino-butanol-(2), Bis-(2-hydroxi-butyl)-amin; 1-Amino-2-methyl-propanol-(2), Bis-(2-hydroxi-2-methyl-propyl)-amin; 2-Amino-2-methyl-propanol-(1), Bis-(monohydroxi-tert.-butyl)-amin; 1-Amino-2-methyl-propanol-(3) und Bis-(3-hydroxi-2-methyl-propyl)-amin oder Mischungen der vorgenannten Amine.

Bevorzugt werden eingesetzt: Ethanolamin, Diethanolamin, 1-Amino-propanol-(2), Bis-(2-hydroxi-propyl)-amin, 1-Amino-propanol(3), Bis-(3-hydroxi-propyl)-amin, Isopropanolamin, Diisopropanolamin oder deren Mischungen, insbesondere Ethanolamin oder Diethanolamin oder deren Mischungen.

Die Alkylenoxide werden vorzugsweise in solchen Mengen eingesetzt, daß auf eine Imino-gruppe der Amidine oder Iminoether etwa 1 Mol und auf eine Aminogruppe der Amidine etwa 2 Mol Alkenoxid entfallen. Von den Alkanolaminen bzw. Dialkanolaminen gelangen vorzugsweise jeweils 1 Mol pro Imino-, Amino- oder Alkylethergruppe der Amidine bzw. Iminoäther zum Einsatz.

Die Umsetzung von Iminoalkylethern mit Aminen zu Amidinen ist im Prinzip literaturbekannt (vgl. Methoden der organischen Chemie, Houben-Weyl, 4.Auflage, (1952) Band 8, Seite 703); ebenso die Hydroxialkylierung von Amidinen mit Alkylenoxiden (vgl. US-PS 2 980 554, Spalte 3, Zeilen 41-43).

Die Herstellung der als Ausgangsmaterialien verwendeten Azo-di-isobuttersäure-iminoalkylether ist ebenfalls bekannt und kann beispielsweise nach dem Verfahren der DE-OS 2 242 520 (Seiten 31-32) erfolgen.

Von den nach den abgehandelten Verfahren hergestellten Azo-di-isobuttersäure-(N,N'-hydroxialkyl)-amidinen seien beispielsweise genannt:
Azo-di-isobuttersäure-(N,N'-bis-2-hydroxiethyl)-amidin,
Azo-di-isobuttersäure-(N,N'-bis-3-hydroxipropyl)-amidin,
Azo-di-isobuttersäure-(N,N'-bis-2-hydroxipropyl)-amidin,
Azo-di-isobuttersäure-(N-2-hydroxiethyl-N'-3-hydroxipropyl)-amidin,
Azo-di-isobuttersäure-(N-2-hydroxiethyl-N'-2-hydroxipropyl)-amidin,

Azo-di-isobuttersäure-(N,N'-bis-3-hydroxibutyl)-amidin,
Azo-di-isobuttersäure-(N,N',N'-tris-2-hydroxiethyl)-amidin,
Azo-di-isobuttersäure-(N,N',N'-tris-3-hydroxipropyl)-amidin,
Azo-di-isobuttersäure-(N,N',N'-tris-2-hydroxipropyl)-amidin,
Azo-di-isobuttersäure-(N-2-hydroxiethyl-N',N'-bis-3-hydroxipropyl)-amidin,
Azo-di-isobuttersäure-(N-2-hydroxiethyl-N',N'-bis-2-hydroxipropyl)-amidin,
Azo-di-isobuttersäure-(N-3-hydroxipropyl-N',N'-bis-2-hydroxiethyl)-amidin,
Azi-di-isobuttersäure-(N-2-hydroxipropyl-N',N'-bis-2-hydroxiethyl)-amidin.

Die unter den angegebenen Reaktionsbedingungen in glatter Reaktion und guten Ausbeuten erhaltenen Azo-di-isobuttersäure-(N,N'-hydroxialkyl)-amidine stellen wasserlösliche, gelbe bis gelborangefarbene Öle dar. Sie sind als Radikalbildner bei Polymerisationsprozessen ungesättigter Verbindungen verwendbar. Sie können ferner bei Vernetzungsprozessen von oder mit ungesättigten Verbindungen bzw. Produkten, gegebenenfalls unter Verschäumung Verwendung finden. Ferner ist ihr Einsatz als Treibmittel bei der Herstellung von Schaumstoffen möglich.

Im folgenden sowie in den Beispielen 7 bis 19 sei die Verwendung der Azo-di-isobuttersäure-(N,N'-hydroxialkyl)-amidine bei der Herstellung wäßriger Polymerisatdispersionen beschrieben.

Polymerisatdispersionen werden vielfach für die Verwendung als Beschichtungsmaterial oder, in Kombination mit Pigmenten und Füllstoffen, als Überzüge für Holz, Metall, Kunststoffe, Keramik und dgl. hergestellt. Für eine gute Haftung der Beschichtungen auf dem Untergrund, auch in feuchter Atmosphäre oder in Gegenwart von Wasser, ist es bedeutsam, daß der Gehalt an wasserlöslichen Salzen in einem Polymerisatfilm möglichst gering ist.

Durch die Salze wird nicht nur die Adhäsion der Filme auf dem Untergrund verschlechtert, sondern auch die Filmauflösung von der Oberfläche her begünstigt. Kritisch ist dies besonders dann, wenn das Polymerisat hart und wenig klebrig ist; geringe Salzmengen stören dann das Zusammenfließen der Latexteilchen besonders empfindlich. Bei Wasserbelastung treten an den Diffusionsgrenzflächen der Latexteilchen osmotische Drucke durch die in Lösung gehenden Salze auf, welche zum Weißanlaufen des Bindemittels und zu seiner Erweichung bis zur Auflösung führen können.

Es wurde daher vorgeschlagen, mit Wasserstoffperoxid oder wasserlöslichen, nicht salzartigen Derivaten des Perhydrols wie tert. Butylhydroperoxid zu polymerisieren. Diese Latices zeigen jedoch eine schlechte Ionen- und Scherstabilität. Weiterhin wurde vielfach angeregt, mit sehr geringen Mengen an Persulfaten zu polymerisieren. Dies führt jedoch zu schlecht reproduzierbaren Ansätzen, welche

gegebenenfalls ganz koagulieren können.

Es wurde nun gefunden, daß man Polymerisatdispersionen ohne Zuhilfenahme anorganischer, die Haftung und Wasserfestigkeit der Polymerisate verschlechternder Salze herstellen kann, wenn man als Polymerisationsinitiatoren anstelle der üblichen Alkali- oder Ammoniumpersulfate oder anderer salzartiger Peroxiverbindungen Azo-di-isobuttersäure-(N,N'-hydroxialkyl)-amidine der Formel I verwendet.

Diese stellen eine wertvolle Ergänzung zu dem schon bekannten wasserlöslichen α,α'-Azo-(α-methyl-γ-sulfo)-buttersäure-dinitril (IV) (vgl. DE-AS 1 111 395), zu den Azodinitrilen vom Typ des α,α'-Azo-(α-methyl-γ-diäthylamino)-buttersäure-dinitrils (V) (vgl. US-PS 2 605 260), zum Typ der γ,γ'-Azo-(γ-cyan)-valeriansäure =α,α'-Azo-(α-methyl-γ-sulfo)-buttersäure-dinitril (VI) (vgl. US-PS 2 520 338) oder schließlich zum 2,2'-Azo-(2-methyl-propion-amidin), (VII), (vgl. US-PS 2 599 299 und 2 599 300) dar.

$$HO_3S-CH_2-CH_2-\underset{C\equiv N}{\overset{CH_3}{C}}-N=N-\underset{C\equiv N}{\overset{CH_3}{C}}-CH_2-CH_2-SO_3H \qquad (IV)$$

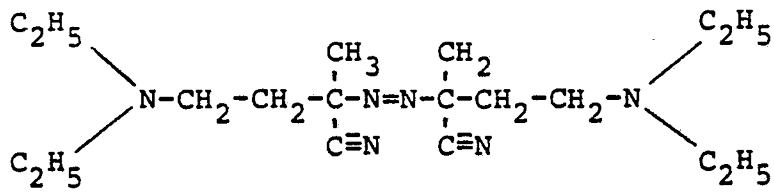

(V)

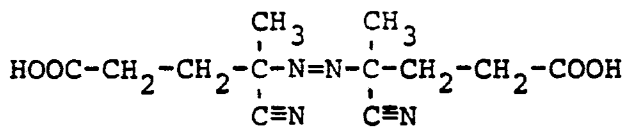

(VI)

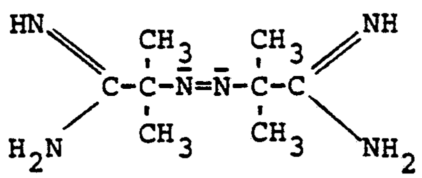

(VII)

Die Amidine vom Typ (VII) werden in der Regel als salzsaure Salze verwendet (vgl. US-PS 2 599 300). Diese müssen jedoch in eisgekühltem Zustand eingesetzt werden, um eine unerwünschte Zersetzung bzw. Hydrolyse zu vermeiden. (vgl. Beispiel zur Herstellung eines Polyethylenlatex mit dem Amidin eines Azodinitrils in: Houben-Weyl, Methoden der Organischen Chemie, 4.Auflage, Band XIV/1 (1961) Seite 222 ff). Chloridionen aber wirken sich in einem für den Korrosionsschutzsektor bestimmten Latex besonders nachteilig aus, da sie die Rostbildung stark beschleunigen. Außerdem können die Amidine vom Typ (VII) nur im neutralen oder sauren Ansatz eine günstige Wirkung entfalten.

Demgegenüber sind die erfindungsgemäßen Initiatoren der Formel (I) in wäßriger Lösung bei Zimmertemperatur stabil. Sie wirken sowohl im sauren als auch alkalischen Medium und sind ausgezeichnet wasserlöslich.

Die Verbindungen vom Typ (VI) sind nur alkalisch oder neutral löslich und für Monomere, welche vorzugsweise im sauren oder schwach sauren Milieu polymerisiert werden sollen, schlecht geeignet.

Gegenüber den Verbindungen vom Typ (V) zeigen die erfindungsgemäßen Initiatoren einen wesentlichen Vorteil: sie besitzen im Molekül freie OH-Gruppen, welche am Anfang und Ende einer Polymerkette eingebaut werden. Diese OH-Gruppen vermitteln eine verbesserte Haftung und sind als reaktive Gruppen für Vernetzungsreaktionen zugänglich und für viele Verwendungszwecke erwünscht.

Die Verbindungen vom Typ (IV) führen zwar zu stabilen Latices; die dann ins Polymerisat eingeführten Sulfogruppen verschlechtern aber andererseits die Wasserfestigkeit der aus solchen Dispersionen herstellbaren Filme. Außerdem müssen die sauren Gruppen der Initiatoren vom Typ (IV) wieder mit Basen abgepuffert werden, so daß sie letztlich keine besonderen Vorteile zu dem sonst üblichen Kalium- oder Ammoniumpersulfat besitzt.

Die erfindungsgemäßen Initiatoren, der Formel (I) können in alkalischem, aber auch im sauren Milieu eingesetzt

werden. Sie führen bereits in niedriger Dosierung zu hohen Polymerisatausbeuten, wie aus den Beispielen hervorgeht.

Es erwies sich als besonders vorteilhaft, die Initiatoren der Formel (I) als Salze bzw. Addukte polymerisierbarer Säuren einzusetzen. Durch diese Maßnahme kann die Polymerisation bei beliebigen pH-Werten von etwa 3 - 9 durchgeführt werden. Als polymerisierbare Säuren seien beispielsweise monoolefinisch ungesättigte Carbonsäuren mit 3-5 C-Atomen genannt wie Acrylsäure, Methacrylsäure, Crotonsäure, Maleinsäure, Itaconsäure; ferner sind Maleinsäurehalbester, Itaconsäurehalbester, Fumarsäurehalbester jeweils mit 1-18 C-Atomen in der Alkoholkomponente geeignet. Gegebenenfalls sind auch Vinylsulfonsäure, Methallylsulfonsäure oder 2-N-Acrylamido-2-methyl-propansulfonsäure zur pH-Wert-Einstellung verwendbar wenn die Elektrolytstabilität der Dispersionen im Vordergrund der Wünsche steht.

Die alkalische Reaktion der Initiatoren der Formel (I) kann schließlich auch durch Zusätze von Alkylsulfonsäuren und/oder Alkylarylsulfonsäuren herabgesetzt werden, wobei Salze mit Emulgatoreigenschaften entstehen. Auch aliphatische Monocarbonsäuren können vorteilhaft verwendet werden.

Die Polymerisation mit den Initiatoren der Formel (I) erfolgt vorzugsweise im Temperaturbereich von 50-90°C,

insbesondere zwischen 50° und 80°C und drucklos bzw. bei Drücken bis 200 bar.

Die Initiatoren können in Mengen von 0,2 - 10 Gew.-%, bezogen auf Gesamtmonomere, eingesetzt werden. Im allgemeinen arbeitet man mit Mengen von 0,3 - 2 Gew.-%. Legt man Wert auf einen erhöhten Einbau von Hydroxylgruppen sowie auf ein niedriges Molekulargewicht, so setzt man entsprechend höhere Mengen ein.

Die Initiatoren können bei der Polymerisation in verschiedenartiger Weise zugegeben werden. Die Dosierung der Initiatormenge kann linear mit einer Geschwindigkeit erfolgen, welche den Zerfall der Initiatoren bei der jeweiligen Polymerisationstemperatur gerade auskompensiert. Man kann aber auch den gesamten Initiator vorlegen oder die Hauptmenge erst zu den letzten Monomerenanteilen hinzugeben. Je nach Art der Initiatordosierung entstehen Produkte unterschiedlicher Molekulargewichtsverteilung und unterschiedlicher Eigenschaften.

Bei Dispersionen, welche als Bindemittel zur Herstellung wäßriger Einbrennlackierungen eingesetzt werden sollen, ist es beispielsweise günstig, die Hauptmenge der OH-Gruppen enthaltenden Initiatoren der Formel (I) erst gegen Ende des Monomerenzulaufs hinzugeben, damit anfangs hochmolekulare, hydroxylgruppenarme, gegen Ende der Polymerisation dagegen niedermolekulare, den Verlauf und Glanz

verbessernde, aufgrund ihres höheren Hydroxylendgruppenanteils gut mit Formaldehydharzen vernetzbare Polymerisatanteile entstehen.

Es hat sich nun überraschenderweise gezeigt, daß die Stabilität der mit den erfindungsgemäßen Initiatoren hergestellten Polymerisatdispersionen sehr gut ist, auch wenn anionische Emulgatoren verwendet werden, obwohl in der Regel beim Einbau kationischer Gruppen in ein Polymerisat bei Anwesenheit anionischer Emulgatoren mit Ausflockungen zu rechnen ist.

Es können also neben den erfindungsgemäßen Polymerisationsinitiatoren die üblichen Emulgatoren der anionischen, der nichtionischen oder der kationischen Klasse eingesetzt werden. Übliche kationische, anionische oder nichtionische Emulgatoren sind beispielsweise beschrieben in Methoden der Organischen Chemie, Houben-Weyl, 4. Auflage (1961), Band XIV/1, Seite 190-208 sowie das gleiche Handbuch 4. Auflage (1959) Band II/2, Seite 113-138 sowie A.M. Schwartz und J.W. Perry, Surface Active Agents, Interscience Publ. Inc., New York, 1958, Seite 25 bis 171. Auch Kombinationen von anionischen mit nichtionogenen im Verhältnis 7 :3 bis 3 : 7 (Molverh.) oder entsprechende Kombinationen kationischer mit nichtionogenen Emulgatoren sind möglich.

Die Polymerisation kann jedoch auch ohne Zuhilfenahme üblicher Emulgatoren durchgeführt werden, wenn man Verbindungen einsetzt, welche emulgatorähnlich wirkende Oligomere bilden oder eine Doppelfunktion als Emulgator und Monomeres spielen.

Solche Stoffe sind beispielsweise Alkali- oder Ammonium- bzw. Aminsalze der Maleinsäurehalbester mit einem Alkoholrest, welcher mehr als 5 C-Atome besitzt, beispielsweise Maleinsäurecyclohexylhalbester-Maleinsäuredodecylhalbester-Salze. Die Polymerisation kann aber auch in Abwesenheit von Emulgatoren unter Zuhilfenahme von Schutzkolloiden, wie beispielsweise Polyvinylalkohol, erfolgen.

An polymerisierbaren Monomeren kommen alle sonst üblicherweise mit Azodiisobuttersäure-Nitril in nichtwäßriger Lösung polymerisierbaren, **olefinisch ungesättigten** Monomeren in Betracht, beispielsweise Styrol, α-Methylstyrol, Butadien, Acrylsäureester mit 1-8 C-Atomen in der Alkoholkomponente, Methacrylsäureester mit 1-8 C-Atomen in der Alkoholkomponente, Acrylnitril, Methacrylnitril, Vinylchlorid, Vinylacetat, Ethylen, Chloropren usw.

Neben den genannten Monomeren können zusätzlich, in geringerem Anteil, wasserlösliche Verbindungen wie Methacrylsäure, Acrylsäure, Maleinsäurehalbester, Itaconsäure und Itaconsäurehalbester, Acrylamid, Methacrylamid usw. einpolymerisiert werden. Ferner können noch funktionelle

Gruppen z.B. OH- oder Epoxigruppen tragenden Comonomere mitverwendet werden, wie ß-Hydroxiethyl(meth)acrylat, ß-Hydroxipropyl(meth)acrylat, Glycidyl(meth)acrylat und N-Methylol- bzw. N-Methylolalkyläther des (Meth)Acrylsäureamids.

Die Polymerisatdispersionen sind vielseitig verwendbar. Die Beispiele 7-19 dienen zur Veranschaulichung der Wirkungsweise der neuen Initiatoren und sollen die Verwendungsmöglichkeit keineswegs einschränken.

Sofern die beschriebenen Dispersionen filmbildend sind, eignen sie sich in ausgezeichneter Weise für Beschichtungen, insbesondere für solche, von denen eine erhöhte Korrosionsschutzwirkung, ein verbesserter Salzsprühtest, eine gute Haftung, eine verbesserte Verträglichkeit und Vernetzung mit Methylol- oder Methyloläthergruppen tragenden Produkte beispielsweise mit Aminoplasten wie Melamin-Formaldehydharzen oder Harnstoff-Formaldehydharzen oder Phenoplasten wie Resolen verlangt wird.

Der Begriff Polymerisate umfaßt in dieser Anmeldung Homo- und Copolymerisate. Unter Copolymerisaten werden nicht nur Copolymerisate mit statistischer Verteilung der einpolymerisierten Monomeren verstanden oder Blockcopolymerisate, sondern auch Pfropfcopolymerisate, bei denen auf ein vorgebildetes Homo- oder Copolymerisat Monomere aufgepfropft worden sind. Statistische Copolymerisate sind von den Copolymerisaten bevorzugt.

Weiter sind Azo-di-isobuttersäure(N,N'-hydroxialkyl)-amidine der Formel (I) ausgezeichnet für die an sich bekannte Polymerisation in homogener Phase geeignet. Darunter wird vorzugsweise die Polymerisation in Lösung und Substanz verstanden. Die Polymerisation kann aber auch lediglich in homogener Phase beginnen und das Polymerisat während der Polymerisation (Fällungspolymerisation) in feinteiliger Form anfallen.

Für die Homo- und Copolymerisation in homogener Phase sind praktisch alle olefinisch ungesättigte Monomeren, die für die Polymerisation mit radikalbildenden Azoverbindungen in Frage kommen,geeignet. Beispielsweise seien genannt:

a) $\alpha$,ß-Monoolefine mit 2 - 8 C-Atomen, wie Äthylen, Propylen, Buten-1, Isobutylen, Diisobutylen;

b) Konjugierte Diolefine mit 4 - 6 C-Atomen, wie Butadien, Isopren, 2,3-Dimethylbutadien, 2-Chlorbutadien, vorzugsweise Butadien;

c) (Meth)Acrylsäure, (Meth)Acrylsäurenitril, (Meth)Acrylsäureamid, (Meth)Acrylsäurealkylester mit 1 - 18, vorzugsweise 1 - 8 C-Atomen in der Alkoholkomponente, wie Methylacrylat, Ethylacrylat, Propylacrylat, Isopropylacrylat, n-Butylacrylat, tert.-Butylacrylat, 2-Ethylhexylacrylat, Stearylacrylat bzw. die entsprechenden Methacrylsäurealkylester, vorzugsweise

Acrylsäure, Acrylnitril, Acrylamid, Methylacrylat, Butylacrylat, tert.-Butylacrylat, 2-Ethylhexylacrylat, Methylmethacrylat;

d) Vinylester organischer Monocarbonsäuren, wobei die Säurekomponente 1 - 18, vorzugsweise 2 - 4 C-Atome enthält, wie Vinylacetat und Vinylpropionat, bevorzugt wird Vinylacetat;

e) Monoolefinisch ungesättigte Halogenkohlenwasserstoffe, wie Vinylchlorid oder Vinylidenchlorid, vorzugsweise Vinylchlorid;

f) Vinylaromaten wie Styrol, o- oder p-Methylstyrol, α-Methylstyrol, α-Methyl-p-isopropylstyrol, α-Methylm-isopropylstyrol, p-Chlorstyrol, vorzugsweise Styrol.

Hierbei werden die weniger polymerisationsfreudigen Monomeren wie α-Methylstyrol und m- bzw. p-Isopropyl-α-methylstyrol, vorzugsweise stets im Gemisch mit mindestens einem weiteren der angegebenen copolymerisierbaren Monomeren eingesetzt.

g) Monoester von α,β-monoolefinisch ungesättigten Monocarbonsäuren mit 3 - 4 C-Atomen mit zweiwertigen gesättigten aliphatischen Alkoholen mit 2 - 4 C-Atomen, wie 2-Hydroxyethylmethacrylat, 2-Hydroxypropylmethacrylat, 4-Hydroxybutylmethacrylat, 2-Hydroxyethylacrylat, 2-Hydroxypropylacrylat, 4-Hydroxybutylacrylat;

h) N-Methylolether des Acrylsäure- und Methacrylsäureamids der allgemeinen Formel VIII

$$CH_2 = \underset{\displaystyle R}{C} - CO - \underset{\displaystyle R_1}{N} - CH_2 - OR_2 \qquad \text{(VIII)}$$

in der

R für Wasserstoff oder Methyl,

$R_1$ für Wasserstoff, Alkyl, Aralkyl oder Aryl,

$R_2$ für Alkyl oder Cycloalkyl, wie Methyl, Ethyl, n-Propyl, Isopropyl, n-Butyl, Isobutyl, Cyclohexyl stehen (vgl. deutsche Auslegeschrift 1 035 363).

Bevorzugt ist der N-Methylolmethylether des Methacrylsäureamids. Die Monomeren der Gruppe h) werden in Mengen von 1 - 20 Gew.-%, bezogen auf Gesamtmonomere, eingesetzt und in das Copolymerisat eingebaut;

i) Di- und Monoester von $\alpha,\beta$-monoolefinisch ungesättigten 3-5 C-Atomen enthaltenden Dicarbonsäuren wie Malein-, Fumar- und Itaconsäure, mit 1 - 18 C-Atomen in der Alkoholkomponente, ferner Maleinsäureanhydrid, Malein- oder Fumarsäure, Amide der Malein- und Fumarsäure, Maleinimide und ungesättigte copolymerisationsfähige Polyester, die als polymerisierbare Bestandteile die Reste

von Malein- und/oder Fumarsäure enthalten. Bevorzugt wird Maleinsäureanhydrid;

j) Vinylalkylether mit 1 - 4 C-Atomen in der Alkylgruppe, wie Vinylmethylether, Vinylethylether, Vinylpropylether, Vinylbutylether.

k) Vernetzend wirkende Monomere mit mehreren nichtkonjugierten olefinisch ungesättigten Kohlenstoff-Kohlenstoff-Bindungen, wie Divinylbenzol, Diallylphthalat, Divinyladipat, Acryl- und/oder Methacrylsäureallylester, Methylenbisacrylamid, Methylenbismethacrylamid, Triallylcyanurat, Triallylisocyanurat, Triacryloylperhydro-S-triazin, Bisacrylate bzw. Bismethacrylate von Glykolen bzw. Polyglykolen mit 2-20 C-Atomen, wie Ethylenglykoldi(meth)-acrylat, Propylenglykoldi-(meth)-acrylat, Butylenglykol-1,4-di-(meth)-acrylat, Tetraethylenglykol-di-(meth)-acrylat, Tris(meth)acrylate des Trimethylolpropans bzw. des Glycerins.

Die vernetzenden Monomeren der Gruppe k) werden bevorzugt in Mengen von 0,1 - 12 Gew.-%, bezogen auf Gesamtmonomere, zur Copolymerisation eingesetzt. In gleichen Anteilen werden sie in das Copolymerisat eingebaut.

Darüber hinaus können auch primäre, sekundäre oder tertiäre Aminoalkylester der (Meth)Acrylsäure mit bevorzugt 2 - 4 C-Atomen in der Alkylgruppe sowie Glycid(meth)acrylat als Comonomere eingesetzt werden und gegebenenfalls über die

Amino- bzw. Epoxidgruppe während oder nach der Copolymerisation vernetzt werden.

Bevorzugt werden Monomere der Gruppen b), c), d), e), f) und i) zur Copolymerisation eingesetzt.

Bei der Polymerisation in Lösung können als Lösungsmittel Wasser und organische Lösungsmittel, beispielsweise Dimethylformamid, tert.-Butanol, Chlorbenzole, usw. verwendet werden.

Die Polymerisation kann entsprechend der Zerfallcharakteristik der erfindungsgemäßen Azoverbindungen zwischen 50°C und 90°C, vorzugsweise 55°C - 75°C, erfolgen. Die Menge des Initiators kann dem angestrebten Molekulargewicht angepaßt werden und zwischen 0,05 bis 10 und mehr Gew.-% der eingesetzten Monomere betragen. Von diesen Angaben über Initiatormenge und Temperatur kann selbstverständlich auch abgewichen werden. Die Polymerisationen in homogener Phase können drucklos bzw. bei Drücken bis zu 1500 bar durchgeführt werden.

Die erhaltenen Polymerisate enthalten am Anfang und Ende jeder Polymerkette in jedem Falle mindestens 2 Hydroxylgruppen eingebaut, herrührend aus den Initiatorbruchstücken.

Die Einführung von Hydroxylgruppen am Anfang und Ende von Polymerketten sind für die verschiedenen Eigenschaften von großer praktischer Bedeutung; einmal können sie aufgrund ihrer Reaktionsfähigkeit mit Verbindungen umgesetzt werden die generell mit Hydroxylgruppen reagieren und weitmaschige Netzwerke bilden. Solche Verbindungen sind beispielsweise Polyisocyanate, Polyepoxide, Polycarbonsäureanhydrid, Methylol- und/oder Methyloläthergruppen enthaltende Verbindungen. Außerdem wird aufgrund der Hydroxylgruppen die Haftung von Polymerisatfilmen erheblich verbessert.

Die Beispiele 20-27 erläutern die Substanz- und Lösungspolymerisation unter Verwendung der Azo-di-isobuttersäure-(N,N'-hydroxialkyl)-amidine der Formel I.

Die in den Beispielen angegebenen Teile und Prozentgehalte beziehen sich auf das Gewicht, sofern nicht bereits vermerkt. Die Grenzviskositätszahl (Intrinsic-Viskosität) $[\eta]$, $[\frac{dl}{g}]$ wurde in den angegebenen Lösungsmitteln bei 25°C gemessen.

Herstellung der Azo-di-isobuttersäure-(N,N'-hydroxialkyl)-amidine

Beispiel 1: Azo-di-isobuttersäure-(N,N'-bis-2-hydroxiethyl)-amidin

114 g (0,5 Mol) Azo-di-isobuttersäure-iminomethylether, 300 ml Methanol und 122 g (2 Mol) Ethanolamin wurden 8 Stunden bei 50°C gerührt. Das Gemisch wurde zur Entfernung von Methanol und Ammoniak (1 Mol) im Wasserstrahlvakuum (12 mbar) bei Temperaturen bis maximal 50°C ausdestilliert. Es blieben 165 g = 88,2 % der Theorie eines gelborangefarbenen, wasserlöslichen Öls, $n_D^{20}$ 1,4880, zurück.

Analyse berechnet für $C_{16}H_{34}N_6O_4$, Molgewicht 374

| | | | | |
|---|---|---|---|---|
| berechnet | C: 51,34 %; | H: 9,09 %; | N: 22,46 %; | O: 17,11 % |
| gefunden | C: 51,5 %; | H: 9,3 %; | N: 22,8 %; | O: 17,4 % |

Beispiel 2: Azo-di-isobuttersäure-(N,N',N'-tris-2-hydroxiethyl)-amidin (Zwei-Stufenverfahren)

In 37,4 g (0,1 Mol) des nach Beispiel 1 erhaltenen Azo-di-isobuttersäure-(N,N'-bis-hydroxiethyl)-amidins wurden unter Kühlung mit Eiswasser bei 20 - 30°C 8,8 g (0,2 Mol) Ethylenoxid eingeleitet. Das Gemisch wurde 5 Stunden bei Zimmertemperatur (ca. 25°C) nachgerührt. Dabei wurden 46 g = 99 % der Theorie eines gelben, zähflüssigen, wasserlöslichen Öls, $n_D^{20}$ 1,4910, erhalten.

Analyse berechnet für $C_{20}H_{42}N_6O_6$, Molgewicht 462

| | | | | |
|---|---|---|---|---|
| berechnet | C: 51,95 %; | H: 9,09 %; | N: 18,18 %; | O: 20,78 % |
| gefunden | C: 52,2 %; | H: 9,2 %; | N: 18,5 %; | O: 20,4 % |

Das gleiche Azo-di-isobuttersäure-(N,N',N'-tris-2-hydroxiethyl)-amidin wird auch nach folgendem Verfahren des Beispiels 3 erhalten:

<u>Beispiel 3:</u> Azo-di-isobuttersäure-(N,N',N'-tris-2-hydroxiethyl)-amidin (Ein-Stufenverfahren)

45,6 g (0,2 Mol) Azo-di-isobuttersäure-iminoethylether, 200 ml Methanol, 42 g (0,4 Mol) Diethanolamin und 26 g (0,4 Mol) Ethanolamin wurden 8 Stunden bei 50°C gerührt. Das Gemisch wurde zur Entfernung von Methanol und Ammoniak (0,4 Mol) im Vakuum (12 mbar) bei Temperaturen bis maximal 50°C ausdestilliert. Es blieben 91 g = 98 % der Theorie eines gelben, zähflüssigen, wasserlöslichen Öls zurück, welches den gleichen Brechungsindex wie das Öl des voranstehenden Beispiels aufwies und mit diesem identisch war.

Beispiel 4: Azo-di-isobuttersäure-(N,N'-bis-2-hydroxipropyl)-amidin

57 g (0,25 Mol) Azo-di-isobuttersäure-iminomethylether, 200 ml Methanol und 75 g (1 Mol) 1-Amino-2-propanol wurden in einem Wasserbad 8 Stunden auf 50°C erwärmt, wobei 0,5 Mol Ammoniak freigesetzt wurde. Das Gemisch wurde bei 50°C zuerst bei 12 mbar, dann bei 0,1 mbar von den flüchtigen Anteilen ausdestilliert. Als Rückstand verblieben 93 g = 87 % der Theorie eines gelblichen, wasserlöslichen Öls, $n_D^{20}$: 1,4691

Analyse berechnet für $C_{20}H_{42}N_6O_4$, Molgewicht 430

| | | | | |
|---|---|---|---|---|
| berechnet | C: 55,81 %; | H: 9,76 %; | N: 19,53 %; | O: 14,88 % |
| gefunden | C: 56,1 %; | H: 10,0 %; | N: 19,7 %; | O: 14,7 % |

Verwendet man anstelle von 1-Amino-2-propanol in dem obigen Ansatz das 1-Amino-3-propanol, so erhält man das Azo-di-isobuttersäure-(N,N'-bis-3-hydroxipropyl)-amidin, als gelbes Öl, welches beim Stehen zu einer gelblichen Paste erstarrt.

Beispiel 5: Azo-di-isobuttersäure-(N,N'-bis-3-hydroxibutyl)-amidin

45,6 g (0,2 Mol) Azo-di-isobuttersäure-iminomethylether, 200 ml Methanol und 73,2 g (0,8 Mol) 1-Amino-3-butanol

wurden 8 Stunden auf 40°C erwärmt, wobei 0,4 Mol Ammoniak freigesetzt wurden. Das Gemisch wurde bei 50°C zuerst bei 12 mbar, dann bei 0,1 mbar von den flüchtigen Anteilen befreit. Als Rückstand verblieben 91 g = 93 % der Theorie, gelbes Öl, $n_D^{20}$: 1,4801

Analyse berechnet für $C_{24}H_{50}N_6O_4$, Molgewicht 486

| | | | | |
|---|---|---|---|---|
| berechnet | C 59,26 %; | H: 10,29 %; | N: 17,28 %; | O: 13,13 % |
| gefunden | C 59,1 %; | H: 10,5 %; | N: 17,4 %; | O: 13,6 % |

<u>Beispiel 6:</u> Azo-di-isobuttersäure-(N-2-hydroxipropyl-N',N'-bis-2-hydroxiethyl)-amidin

128 g (0,5 Mol) Azo-di-isobuttersäure-iminoethylester, 400 ml Methanol, 75 g (1 Mol) 1-Amino-2-propanol und 105 g (1 Mol) Bis-(2-hydroxiethyl)-amin wurden 8 Stunden auf 50° erwärmt, wobei 1 Mol Ammoniak freigesetzt wurde. Das Gemisch wurde bei 50°C/12 mbar und dann bei 50°/0,1 mbar von den flüchtigen Anteilen befreit. Als Rückstand verblieben 224 g = 92 % der Theorie eines gelben, wasserlöslichen Öls, $n_D^{20}$: 1,4780

Analyse berechnet für $C_{22}H_{46}N_6O_6$, Molgewicht 490

| | | | | |
|---|---|---|---|---|
| berechnet | C:53,88 %; | H:9,39 %; | N:17,14 %; | O:19,59 % |
| gefunden | C:53,6 %; | H:9,7 %; | N:17,0 %; | O:19,8 % |

Anwendungsbeispiele 7 bis 19 betreffend die Herstellung wäßriger Polymerisatdispersionen

Beispiel 7

Die Emulsionspolymerisation wird in einem 4 l Fünfhalskolben aus Jenaer Glas mit Dimroth- Rückflußkühler, aufgesetztem Gasblasenzähler (mit zwischen Kühler und Sperrflüssigkeit geschaltetem Dreiwegehahn), einem mit Stickstoffzuleitungshahn versehenen, wassergekühlten Rührer (mit Antriebsmotor und durch Zentrifugalkraft spreizbaren, um 90°C gegeneinander versetzten Rührerflügeln), einem in den Kolben eingeführten Schliffthermometer bzw. einer Thermofühlerhülse, durchgeführt.

Auf die zwei frei verbliebenen Schliffhälse werden zwei Anschützaufsätze gesetzt, welche entweder vier Tropftrichter mit Druckausgleich für die Dosierung der unten angeführten Lösungen I bis IV tragen oder einen Tropftrichter für Lösung I und drei Zulaufstutzen (bestehend aus einer Schliffkappe mit Kern und einem zentral eingeschmolzenen, oben abgebogenen und mit Hahn versehenen Zutropf-Glasrohr) besitzen. An diese absperrbaren Zulaufstutzen werden dann gegebenenfalls die über drei Minidosierpumpen zu den Vorratsgefäßen für die Lösungen bzw. Gemische II, III und IV führenden Schläuche angeschlossen.

In den Kolben füllt man nun die als "Vorlage" bezeichnete Lösung. Nachdem man das Gefäß über den Dreiweghahn (bei geschlossenen Hähnen der Zulaufstutzen und der Stickstoffzuleitung) evakuierte, wird mit Stickstoff ausgeglichen. Durch ein in die $N_2$-Zuleitung geschaltetes, in Wasser eintauchendes T-Rohr mit Rückschlagventil wird ein Überdruck im Glaskolben vermieden.

Das Evakuieren und nachfolgende Begasen mit $N_2$ erfolgt 3 mal. Dann ist alle Luft aus dem Reaktionsraum verdrängt. Die Vorlage wird nun bei leichtem $N_2$-Überdruck (2 Blasen/sec.) unter Rühren (ca. 250 - 300 Upm) auf die gewünschte Polymerisationstemperatur, hier 70°C, aufgeheizt.

Hierbei taucht der Kolben in ein gut isoliertes Wasserbad mit Überlauf, das durch einen Tauchsieder geheizt und über ein Ventil, welches kaltes Wasser einströmen läßt, gekühlt werden kann, wobei sich maximale zeitliche Aufheizrate und maximale Abkühlrate etwa entsprechen.

Tauchsieder und Kühler sind Stellgrößen einer Regeleinrichtung, deren Regelgröße die Innentemperatur (d.h. die Temperatur der Dispersion), deren Störgröße in erster Linie die exotherme Reaktion ist.

Auf diese Weise läßt sich die Innentemperatur sehr genau einstellen. Die Sollwertabweichung ist kleiner als 1 Grad bei gleichmäßiger Reaktion.

Ist nun die gewünschte Polymerisationstemperatur erreicht, wird die Lösung I auf einmal hinzugegeben, wonach die Polymerisation in der Regel sofort anspringt. Sobald ein bläulicher Keimlatex gebildet ist und die Polymerisationswärme abklingt, werden die Lösungen II, III, IV im Verlaufe einer bestimmten Zudosierzeit, hier 6 Stunden, zugetropft oder über geeignete Minidosierer zugepumpt, was genauer ist.

Sind alle Komponenten zudosiert, wird zur Vervollständigung des Monomerenumsatzes noch einige Zeit (hier 2 Stunden) bei einer bestimmten Temperatur (hier 85°C) nachpolymerisiert.

| Vorlage: | g | Gew.-Teile bezogen auf Gesamtkomponenten |
|---|---|---|
| Vollentsalztes oder destilliertes Wasser | 930,0 | 33,646 |
| Natriumlaurylsulfat | 6,0 | 0,217 |
| Acrylsäure-n-butylester | 64,2 | 2,323 |
| Acrylnitril | 17,05 | 0,617 |
| Styrol | 17,05 | 0,617 |
| Methacrylamid | 4,0 | 0,144 |

Lösung I

| | | |
|---|---|---|
| Destilliertes Wasser (bzw. vollentsalztes $H_2O$ | 81,0 | 2,930 |
| Azo-di-isobuttersäure-(N,N'-bis-2-hydroxiethyl)-amidin | 2,5 | 0,090 |
| Methacrylsäure, 50%ig in Wasser | 2,2 | 0,077 |

Lösung II

| | | |
|---|---|---|
| Acrylsäure-n-butylester | 715,2 | 25,875 |
| Acrylnitril | 189,9 | 6,87 |
| Styrol | 189,9 | 6,87 |
| Methacrylamid | 45,7 | 1,6533 |

| Vorlage | g | Gew.-Teile bezogen auf Gesamtkomponente |
|---|---|---|
| **Lösung III** | | |
| Wasser (siehe oben) | 270,0 | 9,768 |
| Azo-di-isobuttersäure (N,N'-bis-2-hydroxiethyl)-amidin | 7,0 | 0,253 |
| Methacrylsäure, 50 %ig in Wasser | 6,2 | 0,224 |
| **Lösung IV** | | |
| Wasser (siehe oben) | 196,0 | 7,091 |
| Natriumlaurylsulfat | 20,2 | 0,731 |
| Summe, gesamt | 2.764,1 | ~ 100 |

Polymerisationstemperatur: 70 °C
Zulaufzeit von II, III, IV 6 Stunden
Nachpolymerisiert: 2 Stunden bei 85 °C.

Der auf diese Weise erhaltene, dünnflüssige Latex besitzt einen Feststoffgehalt von 45 - 46 % und läßt sich leicht durch ein 30 µ-Tuch aus Perlongewebe mit quadratischen Maschen passieren, wobei nur wenig grobteiliges Koagulat (ca. 0,5 - 5 g) zurückbleibt.

Der Latex besitzt einheitliche Teilchen von ca. 130 nm Durchmesser. Er trocknet bei 25°C zu klaren, klebfreien Filmen mit guter Wasserfestigkeit auf.

Ein auf die Filmoberfläche gesetzter Wassertropfen führt nach ca. 30 Minuten zu keinerlei Trübung oder Filmauflösung. Zur Verbesserung der Ionenbeständigkeit kann der Latex nachträglich noch mit nichtionischen Emulgatoren nachbehandelt werden. Dies ist indessen nur bei speziellen Anwendungen nötig.

Der Latex läßt sich mit handelsüblichen, für Einbrennlacke verwendeten wasserlöslichen Melamin-Formaldehyd-Harzen oder Harnstoffen-Formaldehydharzen vermischen. Diesen Mischungen können ferner Pigmente und Füllstoffe zugesetzt werden. Durch die Abwesenheit üblicher anorganischer Salze zeigen solche wäßrigen Einbrennsysteme eine verbesserte Wasserfestigkeit und Haftung auf verschiedenen Untergründen, insbesondere auf Metallen.

Das dem Latex zugrunde liegende Polymerisat ist gelfrei in Tetrahydrofuran oder Dimethylformamid löslich und besitzt eine Grenzviskositätszahl von $[\eta] = 3{,}0$ dl/g bei 25°C in Tetrahydrofuran.

Es besteht aus:

62,7 % polymerisierten Acrylsäurebutylestereinheiten
16,65 % Acrylnitrileinheiten
16,65 % Styroleinheiten
4,0 % Methacrylamideinheiten

Beispiel 8: (Vergleiche)

A) Man arbeitet wie in Beispiel 7, ersetzt jedoch den Initiator durch die gleiche Menge Ammoniumperoxidsulfat. Der entstehende, koagulatfreie Latex ist gelb verfärbt. Klarfilme aus diesem Latex zeigen eine schlechtere Haftung auf Glas und eine größere Wasserempfindlichkeit. Mit diesem Latex zubereitete Melamin-Formaldehyd-Harz-Abmischungen ergeben in analoger Kombination mit Pigmenten deutlich schlechtere Ergebnisse bei der Wasserlagerung. Die auf Metall eingebrannten Schichten lösen sich vom Untergrund ab.

b) Beispiel 7 wird wiederholt mit der Änderung, daß der Initiator durch die gleiche Menge γ, γ'-Azo-(γ-cyan)-valeriansäure (Formel (VI), gelöst in der äquivalenten Menge verdünnter, wäßriger 10 %iger Ammoniaklösung. Der Latex besitzt eine Teilchengröße von ca. 150 nm, ist stark gelb gefärbt und enthält ca. 15 g Koagulat. Der bei 25°C aufgetrocknete klare Film, auf dessen Oberfläche ein Wassertropfen mit einer Pipette aufgebracht wurde, trübt sich und führt nach ca. 30 Minuten zur Filmauflösung.

Beispiel 9

Man arbeitet wie in Beispiel 7, ersetzt jedoch den Initiator durch Azo-di-isobuttersäure-(N,N',N'-tris-2-hydroxiethyl)-amidin. Man erhält einen nahezu monodispersen Latex mit ähnlichen Eigenschaften wie dem in Beispiel 7 beschriebenen Typ.

Beispiel 10

In der in Beispiel 7 beschriebenen Apparatur wird die unten als "Vorlage" bezeichnete Lösung eingefüllt und unter Stickstoff auf 75°C aufgeheizt. Nach Injektion von I erfolgt die Zudosierung von II, III und IV im Verlauf von 5 Stunden, wonach die Temperatur auf 80°C erhöht und 2 Stunden nachgerührt wird.

Es resultiert ein dünnflüssiger, koagulatfreier Latex mit einem Feststoffgehalt von 46,5 %. Nach dem Entfernen der Restmonomeren kann der Latex mit wasserlöslichen, handelsüblichen Harnstoff-Formaldehydharzen oder Melamin-Formaldehydharzen und Pigmenten abgemischt und als wäßriger Einbrennlack verwendet werden.

| Vorlage | g | Gew.-Teile bezogen auf Gesamtkomponenten |
|---|---|---|
| Destilliertes Wasser | 919,0 | 31,8971 |
| Natriumlaurylsulfat | 6,0 | 0,208 |
| Acrylsäure-n-butylester | 60,0 | 2,082 |
| Acrylnitril | 15,0 | 0,5205 |
| Styrol | 15,0 | 0,5205 |
| Methacrylsäure-2-hydroxipropylester | 10,0 | 0,347 |
| Methacrylamid | 2,0 | 0,0694 |
| Methacrylsäure | 2,0 | 0,0694 |

Lösung I

| | | |
|---|---|---|
| Destilliertes Wasser | 100,0 | 3,471 |
| Azo-di-isobuttersäure-(N,N'-bis-2-hydroxiethyl)-amidin | 3,0 | 0,104 |
| Methacrylsäure 50 %ig in $H_2O$ | 2,64 | 0,0916 |

Lösung II

| | | |
|---|---|---|
| Acrylsäure-n-butylester | 700,0 | 24,296 |
| Acrylnitril | 175,0 | 6,074 |
| Styrol | 175,0 | 6,074 |
| Methacrylsäure-2-hydroxipropylester | 116,7 | 4,050 |
| Methacrylamid | 23,3 | 0,809 |
| Methacrylsäure | 23,3 | 0,809 |

| Vorlage | g | Gew-Teile bezogen auf Gesamtkomponenten |
|---|---|---|
| Lösung III | | |
| Destilliertes Wasser | 300,0 | 10,412 |
| Azo-di-isobuttersäure-(N,N'-bis-2-hydroxi-ethyl)-amidin | 7,0 | 0,243 |
| Methacrylsäure, 50%ig in Wasser | 6,2 | 0,215 |
| Lösung IV | | |
| Destilliertes Wasser | 200,0 | 6,941 |
| Natriumlaurylsulfat | 20,0 | 0,694 |
| Summe, gesamt | 2.881,14 | 100 |

Polymerisationstemperatur: 75°C

Zulaufzeit von II,III,IV: 5 h

Nachpolymerisiert: 2 h bei 80°C

Da die Copolymerisation der Monomeren wie auch in den nachfolgenden Beispielen nahezu quantitativ erfolgte, entspricht die integrale Zusammensetzung des Polymerisats der Zusammensetzung des Monomerengemisches:

| | | |
|---|---|---|
| 57,7 | Gew.-% | Acrylsäurebutylestereinheiten |
| 14,45 | Gew.-% | Acrylnitrileinheiten |
| 14,45 | Gew.-% | Styroleinheiten |
| 9,6 | Gew.-% | Methacrylsäure-2-hydroxipropylestereinheiten |
| 1,9 | Gew.-% | Methacrylsäureeinheiten |
| 1,9 | Gew.-% | Methacrylamideinheiten |
| 100 | Gew.-% | |

Beispiel 11

In einem Dreihalskolben werden

| | | |
|---|---|---|
| 400 | Gew.-Teile | destilliertes Wasser |
| 1 | Gew.-Teil | eines Alkylmonosulfonates mit 12 bis 14 C-Atomen |
| 0,25 | Gew.-Teile | Azo-di-isobuttersäure-(N,N'-bis-2-hydroxiethyl)-amidin |

vorgelegt und auf 80°C erhitzt.

Sodann werden 50 Gew.-Teile einer Mischung aus

200 Gew.-Teile Styrol
275 Gew.-Teile Acrylsäure-n-butylester
25 Gew.-Teile Methacrylsäure

zugegeben. Nachdem 30 Minuten bei 80°C gerührt wurde, tropft man innerhalb von 3 Stunden gleichmäßig den Rest der Monomerenmischung und eine Lösung aus

| | | |
|---|---|---|
| | 350 Gew.-Teilen | destilliertem Wasser |
| | 10 Gew.-Teile | eines Alkylmonosulfonates mit 12 bis 14 C-Atomen |
| und | 5 Gew.-Teile | Azo-di-isobuttersäure-(N,N'-bis-hydroxiethyl)-amidin |

bei 80°C zu. Es wird 2 Stunden bei 80°C nachgerührt. Nach der Entgasung erhält man 1230 Gew.-Teile einer koagulatfreien Dispersion mit einem Feststoffgehalt von 39 % und einer mittleren Teilchengröße von 145 nm. Die Auslaufzeit im DIN-Becher (2 mm Düse) beträgt 69 s.

**Beispiel 12**

Gemäß Beispiel 11 wird anstelle der dort angegebenen Monomerenmischung eine Mischung aus

190 Gew.-Teilen Styrol
260 Gew.-Teilen Acrylsäure-n-butylester
25 Gew.-Teilen Methacrylsäure
und 25 Gew.-Teilen 2-Hydroxipropyl-methacrylat

eingesetzt. Alle anderen Bedingungen bleiben unverändert. Nach der Entgasung erhält man 1,150 Gew.-Teile einer koagulatfreien Dispersion mit einem Feststoffanteil von 39,5 % und einer mittleren Teilchengröße von 138 nm.

Die Auslaufzeit im DIN-Becher (2 mm Düse) beträgt 78 s. Die so erhaltene Dispersion trocknet bei 25°C zu klaren, wasserfesten Filmen auf. Sie läßt sich mit Melamin-Formaldehydharzen bzw. Harnstoff-Formaldehydharzen und Pigmenten abmischen. Diese Abmischungen lassen sich auf Metallen einbrennen, wobei gut haftende wasserfeste und lösungsmittelfeste Überzüge resultieren.

Beispiel 13

Gemäß Beispiel 12 wird als Initiator eine äquivalente Menge Azo-di-isobuttersäure-(N,N'-bis-2-hydroxipropyl)-amidin eingesetzt. Nach der Entgasung erhält man 1190 Gew.-Teile einer koagulatfreine Dispersion mit einem Feststoffanteil von 37,5 % und einer mittleren Teilchengröße von 132 nm. Die Auslaufzeit im DIN-Becher (2 mm-Düse) beträgt 75 s.

Beispiel 14

Polyvinylchloridlatex

In einem Autoklaven aus rostfreiem Stahl mit Ankerrührer werden

| | | | |
|---|---|---|---|
| | 3000 | Gew.-Teile | destilliertes Wasser |
| | 7,5 | Gew.-Teile | eines Alkylmonosulfonates mit 12 bis 14 C-Atomen |
| | 6 | Gew.-Teile | Azo-di-isobuttersäure (N,N',N'-tris-2-hydroxiethyl)-amidin |
| und | 10 | Gew.-Teile | Essigsäure |

vorgelegt. Der Autoklav wird evakuiert, zweimal mit 3 bar Stickstoff gespült und wiederum evakuiert. In den Autoklaven werden nun

1500 Gew.-Teile Vinylchlorid

gepumpt und die Innentemperatur auf 65°C gebracht. Diese Temperatur wird 12 Stunden gehalten. Der Anfangsdruck betrug 13 bar und der Druck am Ende der Polymerisation 4 bar.

Nach der Entgasung erhält man 4175 Gew.-Teile an koagulatfreiem Latex mit einer Teilchengröße von 180 nm. Das Polymerisat hat eine Grenzviskositätszahl von 0,77 (gemessen in Tetrahydrofuran). Der Feststoffanteil beträgt 29 % und die Auslaufzeit im DIN-Becher (2 mm Düse) 75 s.

Beispiel 15

Polyvinylacetatlatex

In einem Dreihalskolben bereitet man eine Lösung aus

| | | | |
|---|---|---|---|
| | 12,8 | Gew.-Teilen | Polyvinylalkohol 2) |
| in | 125 | Gew.-Teilen | destilliertes Wasser |

In die auf 68°C erhitzte Lösung tropft man innerhalb von 3 1/2 Stunden gleichzeitig

1. Eine Lösung von

| | | | |
|---|---|---|---|
| | 0,35 | Gew.-Teilen | Azo-di-isobuttersäure-(N,N'-bis-2-hydroxiethyl)-amidin |
| in | 40 | Gew.-Teilen | destilliertem Wasser |
| und | 0,8 | Gew.-Teilen | Essigsäure |

2. 191 Gew.-Teile Vinylacetat

Während des Eintropfens wird die Temperatur konstant auf 68°C gehalten. Anschließend rührt man weitere 3 1/2 Stunden bei 68°C und tropft dann innerhalb von 15 Minuten eine Lösung von

| | | | |
|---|---|---|---|
| | 0,05 | Gew.-Teilen | Azo-di-isobuttersäure-(N,N'-bis-2-hydroxiethyl)-amidin |
| in | 10 | Gew.-Teilen | destilliertem Wasser |

ein. Man rührt 45 Minuten bei 90°C.

2) Teilverseiftes Polyvinylacetat (Hydrolysegrad 88 %)

Nach der Entgasung erhält man 350 Gew.-Teile einer hochviskosen Dispersion mit einem Feststoffanteil von 51 %. Die mittlere Teilchengröße beträgt 260 nm.

Beispiel 16

Gemäß Beispiel 15 wird als Initiator eine

äquivalente Menge Azo-di-isobuttersäure (N,N',N'-tris-2-hydroxiethyl)-amidin

eingesetzt.

Es werden 342 Gew.-Teile einer hochviskosen Dispersion mit einem Feststoffanteil von 48 % und einer Teilchengröße von 245 nm erhalten.

Beispiel 17

Polychloroprenlatex

In einem Dreihalskolben werden

120 Gew.-Teile destilliertes Wasser
5 Gew.-Teile Na-Salz einer disproportionierten Abietinsäure
und 0,6 Gew.-Teile Natriumhydroxid

vorgelegt. Nachdem 30 Minuten mit Stickstoff bei Raumtemperatur gespült wurde, werden

100 Gew.-Teile eines mit 180 ppm Phenothiazin stabilisierten Chloroprens

eingerührt. Unter weiterer Stickstoffspülung erhitzt man den Kolbeninhalt auf 64°C und tropft sodann innerhalb von 2 Stunden eine Lösung von

2,5 Gew.-Teilen Azo-di-isobuttersäure (N,N'-bis-2-hydroxiethyl)-amidin
in 100 Gew.-Teilen destilliertem Wasser zu.

Es wird 3 Stunden bei 64°C nachgerührt. Das nicht umgesetzte Chloropren wird durch Wasserdampfdestillation entfernt.

Man erhält 310 Gew.-Teile einer stabilen Dispersion mit einem Feststoffgehalt von 25,5 % und einer mittleren Teilchengröße von 185 nm.

Beispiel 18

Gemäß Beispiel 17 wird als Initiator eine äquivalente Menge Azo-di-isobuttersäure-(N,N',N'-tris-2-hydroxiethyl)-amidin eingesetzt.

Nach Entfernung des nicht umgesetzten Chloroprens durch Wasserdampfdestillation erhält man 290 Gew.-Teile einer koagulatfreien Dispersion mit einem Feststoffanteil von 27 % und einer mittleren Teilchengröße von 170 nm.

**Beispiel 19**

**Styrol-Butadien Latex**

In einem Autoklaven aus rostfreiem Stahl mit Ankerrührer werden

| | | |
|---|---|---|
| 2700 | Gew.-Teile | destilliertes Wasser |
| 70 | Gew.-Teile | Na-Salz einer disproportionierten Abietinsäure |
| 7,5 | Gew.-Teile | n-Dodecylmercaptan |
| 6 | Gew.-Teile | Azo-di-isobuttersäure-(N,N'-bis-2-hydroxiethyl)-amidin |

vorgelegt. Der Autoklav wird evakuiert, 2 mal mit 3 bar Stickstoff gespült, wiederum evakuiert und sodann werden nacheinander

435 Gew.-Teile Styrol
und 1065 Gew.-Teile Butadien

eingepumpt. Bei einer Rührerdrehzahl von 150 U.Min.$^{-1}$ wird der Inhalt auf 65°C geheizt, wobei der Druck 10,5 bar beträgt und diese Temperatur 10 Stunden gehalten. Der Druck beträgt jetzt 8,0 bar. Nach beendeter Reaktion wird zur Stabilisierung eine Lösung von

1 Gew.-Teil Hydrochinon
in 50 Gew.-Teilen destilliertem Wasser

eingedrückt.

Nach der Entgasung erhält man 3450 Gew.-Teile eines koagulatfreien Latex mit einem Feststoffanteil von 33 % und einer mittleren Teilchengröße von 190 nm. Die Auslaufzeit im DIN-Becher (2 mm Düse) beträgt 130 s.

Substanz- und Lösungspolymerisation (Beispiel 20-26)

Beispiel 20

In einem Bombenrohr aus Glas wird eine Lösung von

0,6 Gew.-Teilen Azo-di-isobuttersäure-(N,N',N'-tris-2-hydroxiethyl)-amidin
in 30 Gew.-Teilen Styrol

zur Entfernung des Luftsauerstoffs 3 Minuten mit Stickstoff begast. Nach dem Zuschmelzen des Bombenrohres wird 8 Stunden auf 75°C erhitzt.

Der polymerisierte Inhalt wird in 300 Gew.-Teilen Tetrahydrofuran gelöst und anschließend mit der 10-fachen Gewichtsmenge Methanol ausgefällt. Nach einer Vakuumtrocknung bei 50°C werden 17 Gew.-Teile an gereinigtem Polymerisat erhalten.

Grenzviskositätszahl (gemessen in Tetrahydrofuran): 0,49

Über die eingebauten endständigen Hydroxylgruppen lassen sich die Polymerisate mit Di- und Polyioscyanaten vernetzen.

2 Gew.-Teile des nach Beispiel 20 erhaltenen Polystyrols mit endständigen Hydroxylgruppen werden in 18 Gew.-Teilen wasserfreiem Chlorbenzol gelöst. Die Lösung wird in Gegenwart von 0,05 Gew.-Teilen Zinn-II-octoat mit 0,2 Gew.-Teilen Hexamethylendiisocyanat vernetzt.

Nach 24-stündigem Stehen bei Raumtemperatur hat sich ein vernetztes Gel gebildet. Ein direkt nach der Abmischung auf Glas ausgegossener Film ist nach 24-stündiger Austrocknung ebenfalls vernetzt.

<u>Beispiel 21</u>

Nach der gleichen Arbeitsweise wie in Beispiel 20 wird eine Lösung von

0,6 Gew.-Teilen Azo-di-isobuttersäure-(N,N',N'-tris-2-hydroxiethyl)-amidin
in 30 Gew.-Teilen Methacrylsäuremethylester

polymerisiert. Nach der Umfällung werden 19 g Polymerisat mit einer Grenzviskositätszahl in Tetrahydrofuran von 0,45 erhalten.

Beispiel 22

In einem Dreihalskolben mit Thermometer, Rückflußkühler und Stickstoffbegasung wird eine Lösung aus

30 Gew.-Teilen Acrylnitril
70 Gew.-Teilen Dimethylformamid
und 0,3 Gew.-Teilen Azo-di-isobuttersäure-(N,N'-bis-2-hydroxiethyl)-amidin

6 Stunden bei 80°C gerührt. Die entstandene hochviskose Lösung wird in 1000 Gew.-Teilen Wasser gefällt und bei 50°C im Vakuum getrocknet.

Man erhält 15 Gew.-Teile Polymerisat mit einer Grenzviskositätszahl (gemessen in Dimethylformamid) von 0,58.

Beispiel 23

Gemäß Beispiel 22 wird eine Lösung aus

30 Gew.-Teilen Vinylacetat
70 Gew.-Teilen tert.-Butanol
0,3 Gew.-Teilen Azo-di-isobuttersäure-(N,N'-bis-2-hydroxiethyl)-amidin
und 2 Gew.-Teilen Essigsäure

6 Stunden bei 80°C gerührt. Nach Ausfällung der hochviskosen Lösung in 1000 Gew.-Teilen Wasser erhält man nach der Trocknung 13 Gew.-Teile Polymerisat mit einer Grenzviskositätszahl (gemessen in Dimethylformamid) von 0,35.

Beispiel 24

In einem Dreihalskolben werden

500 Gew.-Teile destilliertes Wasser

vorgelegt. Unter laufender Stickstoffspülung wird auf eine Innentemperatur von 70°C geheizt und sodann innerhalb von 2 Stunden gleichmäßig zudosiert:

a) 100 Gew.-Teile Acrylnitril

b) Eine Lösung von
0,5 Gew.-Teilen Azo-di-isobuttersäure-(N,N'-bis-2-hydroxiethyl)-amidin
in 50 Gew.-Teilen destilliertem Wasser

Nach beendetem Zutropfen wird noch 2 Stunden bei 70°C nachgerührt. Das ausgefallene Polyacrylnitril wird abgesaugt, gut mit Wasser gewaschen und bei 50°C getrocknet.

Man erhält 75 Gew.-Teile Polymerisat.

Beispiel 25

Unter gleichen Versuchsbedingungen wie bei Beispiel 24 werden als Initiator 0,6 Gew.-Teile Azo-di-isobuttersäure-(N,N'-bis-2-hydroxipropyl)-amidin eingesetzt.

Man erhält 62 Gew.-Teile Polymerisat.

Beispiel 26

Wie in Beispiel 20 wird eine Mischung aus 22,5 Teilen Styrol und 7,5 Teilen Acrylnitril in Gegenwart von 0,15 Teilen

Azo-di-isobuttersäure-(N,N'-bis-2-hydroxiethyl)-amidin polymerisiert. Anstelle von Tetrahydrofuran wird Dimethylformamid als Lösungsmittel für das Copolymerisat verwendet. Nach der Vakuumtrocknung werden 19 Teile gereinigtes Copolymerisat aus 72 % Styrol- und 28 % Acrylnitrileinheiten erhalten, das, gemessen in Dimethylformamid eine Grenzviskositätszahl von 0,86 besitzt.

Beispiel 27

Eine Mischung aus 45 Teilen Styrol und 55 Teilen n-Butylacrylat, 400 Teilen Chlorbenzol und 2 Teilen Azo-di-isobuttersäure-(N,N'-bis-3-hydroxibutyl)-amidin werden wie in Beispiel 22 jedoch 6 Stunden bei 75°C polymerisiert. Das entstandene Copolymerisat wird aus seiner Chlorbenzollösung mit 1500 Teilen Methanol gefällt und bei 50°C im Vakuum getrocknet. Man erhält 65 Teile Copolymerisat aus 49 % Styrol- und 51 % n-Butylacrylateinheiten mit einer Grenzviskositätszahl (gemessen Dimethylformamid) von 0,72.

Vernetzung des Copolymerisats über die eingebauten OH-Gruppen

2 Teile des Copolymerisats, gelöst in 8 Teilen wasserfreiem Chlorbenzol, werden mit 0,05 Teilen Zinn-II-octoat

und 0,2 Teilen Isophorondiisocyanat vermischt und von dieser Lösung Filme auf Glas gezogen. Nach 24 Stunden bei Raumtemperatur sind die Filme vernetzt und können nicht mehr durch Chlorbenzol gelöst werden.

Patentansprüche

1. Azo-di-isobuttersäure-(N,N'-hydroxialkyl)-amidine der Formel

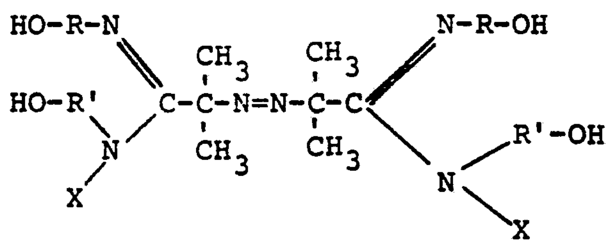

worin

R und R' lineare oder verzweigte Alkylenreste mit 2 bis 4 C-Atomen und

X R'-OH oder H bedeuten.

2. Verfahren zur Herstellung von Azo-di-isobuttersäure-(N,N'-hydroxialkyl)-amidinen gemäß Anspruch 1, dadurch gekennzeichnet, daß man Azo-di-isobuttersäure-iminoalkylether mit 1-4 C-Atomen in der Alkylgruppe mit mindestens einem Monoalkanolamin mit 2-4 C-Atomen oder mit einem Gemisch aus mindestens einem Mono- und mindestens einem Dialkanolamin mit jeweils 2-4 C-Atomen in einem Alkanolrest, wobei im Gemisch das Molverhältnis von Mono- zu Dialkanolaminen 1:1 beträgt, bei Temperaturen von etwa 0 bis 50°C in etwa äquivalenten Mengenverhältnissen umsetzt.

3. Verfahren nach Anspruch 2, dadurch gekennzeichnet, daß man anstelle von Azo-di-isobuttersäure-iminoalkylethern mit 1-4 C-Atomen Azo-di-isobuttersäureamidin einsetzt oder ein Azo-di-isobuttersäureamidin, dessen an die Stickstoffatome der beiden Amidinreste gebundenen Wasserstoffatome teilweise durch Hydroalkylreste mit 2-4 C-Atomen substituiert sind.

4. Verfahren nach Anspruch 2, dadurch gekennzeichnet, daß man nach der Kondensation des Azo-di-isobuttersäureiminoalkylethers (1 Mol) mit einem Monoalkanolamin (4 Mol) zum Azo-di-isobuttersäure-(N,N'-bis-hydroxialkyl)-amidin letzteres mit 2 Mol eines Alkylenoxids mit 2-4 C-Atomen zu einem Azo-di-isobuttersäure-(N,N',N'-tris-hydroxialkyl)-amidin umsetzt.

5. Verwendung der Azo-di-isobuttersäure-(N,N'-hydroxialkyl)-amidine gemäß Anspruch 1 als Radikalbildner bei Polymerisationsprozessen ungesättigter Verbindungen und/oder bei Vernetzungsprozessen von oder mit mehrfach ungesättigten Produkten, gegebenenfalls unter Verschäumung.

6. Verwendung der Azo-di-isobuttersäure-(N,N'-hydroxialkyl)-amidine gemäß Anspruch 1 als Radikalbildner bei der Polymerisation in Masse oder Lösung.

7. Verwendung der Azo-di-isobuttersäure-(N,N'-hydroxialkyl)-amidine gemäß Anspruch 1 als Treibmittel bei der Herstellung von Schaumstoffen.

Europäisches Patentamt

# EUROPÄISCHER RECHERCHENBERICHT

0009186

Nummer der Anmeldung

EP 79 10 3332

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | US - A - 2 599 299 (R.W. UPSON)<br>* Beispiel 6 *<br>-- | 1 |
| D,A | DE - A - 1 693 164 (BAYER) | |
| D,A | DE - A - 2 242 520 (BAYER)<br>---- | |

KLASSIFIKATION DER ANMELDUNG (Int.Cl. 3)

C 07 C 123/00
C 08 F 4/04
C 08 J 9/10
C 08 K 5/31

RECHERCHIERTE SACHGEBIETE (Int. Cl. 3)

C 07 C 123/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 17-12-1979 | DAUKSCH |